(19) Europäisches Patentamt
European Patent Office
Office européen des brevets

(11) **EP 2 442 675 B1**

(12) **EUROPEAN PATENT SPECIFICATION**

(45) Date of publication and mention
of the grant of the patent:
**21.05.2014 Bulletin 2014/21**

(51) Int Cl.:
*A23L 1/29* *(2006.01)*     *A23L 1/305* *(2006.01)*
*A23L 1/22* *(2006.01)*     *A23L 1/30* *(2006.01)*

(21) Application number: **11705722.4**

(22) Date of filing: **28.01.2011**

(86) International application number:
**PCT/US2011/022928**

(87) International publication number:
**WO 2011/094544 (04.08.2011 Gazette 2011/31)**

(54) **NUTRITIONAL EMULSIONS COMPRISING CALCIUM HMB AND SOLUBLE PROTEIN**

NAHRUNGSEMULSIONEN MIT CALCIUM-HMB UND LÖSLICHEM PROTEIN

ÉMULSIONS NUTRITIONNELLES COMPRENANT CALCIUM-HMB ET UNE PROTÉINE SOLUBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2010 US 299567 P**

(43) Date of publication of application:
**25.04.2012 Bulletin 2012/17**

(73) Proprietor: **Abbott Laboratories
Abbott Park, IL 60064-6008 (US)**

(72) Inventors:
• **JOHNS, Paul, W
Columbus, Ohio 43221 (US)**
• **KENSLER, Ann
Sugar Grove, Ohio 43155 (US)**

(74) Representative: **Modiano, Micaela Nadia et al
Modiano & Partners
Thierschstrasse 11
80538 München (DE)**

(56) References cited:
**DE-U1- 29 709 313     US-A- 5 028 440
US-A- 5 726 146**

• **KREIDER R B ET AL: "EFFECT OF CALCIUM
BETA-HYDROXY-BETA-METHYLBUTYRATE
(HMB) SUPPLEMENTATION DURING
RESISTANCE-TRAINING ON MARKERS OF
CATABOLISM, BODY COMPOSITION AND
STRENGTH", INTERNATIONAL JOURNAL OF
SPORTS MEDICINE, THIEME, STUTTGART, DE,
vol. 20, no. 8, 1 November 1999 (1999-11-01),
pages 503-509, XP008037671, ISSN: 0172-4622,
DOI: DOI:10.1055/S-1999-8835**

EP 2 442 675 B1

**Description**

## FIELD OF THE DISCLOSURE

The present disclosure relates to nutritional emulsions comprising calcium beta-hydroxy-beta-methylbutyrate (calcium HMB) and soluble protein.

## BACKGROUND OF THE DISCLOSURE

Beta-hydroxy-beta-methylbutyrate (HMB) is a naturally occurring amino acid metabolite that is often formulated into a variety of nutritional products and supplements. HMB is commonly used in such products to help build or maintain healthy muscle mass and strength in selected individuals.

HMB is a metabolite of the essential amino acid leucine and has been shown to modulate protein turnover and inhibit proteolysis. In most individuals, muscle converts approximately 5% of available leucine to HMB, thus producing about 0.2 to 0.4 grams of HMB per day for a 70 kg male. In studies where various kinds of stress were induced in animals, HMB supplementation increased lean mass. Clinical studies also suggest that HMB has at least two functions in recovery from illness or injury including protection of lean mass from stress-related damage and enhancement of protein-synthesis. It has been suggested that HMB may also be useful in enhancing immune function, reducing the incidence or severity of allergy or asthma, reducing total serum cholesterol and low density lipoprotein cholesterol, increasing the aerobic capacity of muscle, and other uses.

Since HMB is most often used in individuals to support the development and maintenance of healthy muscle mass and strength, many HMB products have been formulated with additional nutrients that may also be helpful in promoting healthy muscle. Some of these HMB products contain additional nutrients such as fat, carbohydrate, protein, vitamins, minerals and so forth. Calcium HMB is the most commonly used form of HMB when formulated into oral nutritional products, which products include tablets, capsules, reconstitutable powders, and nutritional liquids and emulsions. Nutritional emulsions are particularly useful in this regard because such emulsions may contain a balance of fat, protein, carbohydrates, vitamins, and minerals that is useful for helping maintain healthy muscle.

It has no been found, however, that nutritional emulsions containing calcium HMB are often not physically stable over time, that such emulsions are not readily stable with many protein systems, and that protein-containing and or other sediments form in the emulsions during or after formulation, especially when packaged and stored for extended periods of time.

It has also been discovered that these nutritional emulsions containing calcium HMB develop an undesirably bitter off flavor or after taste after the emulsion has been packaged and stored for extended periods of at least 1-3 months.

There is therefore a need for nutritional emulsions comprising calcium HMB that remain physically stable during shelf life and or do not develop a bitter flavor or after taste over time.

## SUMMARY OF THE DISCLOSURE

One embodiment of the present disclosure is directed to a nutritional emulsion comprising fat, carbohydrate, protein and calcium HMB, wherein the protein comprises from about 50% to 100% by weight of soluble protein. The soluble protein is preferably selected from sodium caseinate, whey protein concentrate, and combinations thereof. Sodium caseinate is most preferred.

Another embodiment of the present disclosure is directed to nutritional emulsions comprising fat, carbohydrate, protein, and calcium HMB, wherein the protein comprises from about 50% to 100% by weight of soluble protein and the nutritional emulsion has a weight ratio of soluble protein to calcium HMB of from about 5:1 to about 12:1.

Another embodiment of the present disclosure is directed to a packaged nutritional composition comprising a hermetically sealed container and a nutritional emulsion contained therein, the nutritional emulsion comprising fat, carbohydrate, protein and calcium HMB, wherein the protein comprises from about 50% to about 100% by weight of soluble protein and the packaged composition has been stored for between 3 and 24 months at 18°C to 24°C for between 3 and 24 months following formulation and packaging of the packaged composition.

It has been found that the addition of calcium HMB to nutritional emulsions can result in the development of a bitter flavor or after taste, which typically does not manifest until the product is manufactured, packaged, and stored for a period of at least about 1 to about 3 months. Indeed, it has been found that nutritional emulsions comprising calcium HMB often produce little or no bitter flavor or after taste when consumed within about 1 month, including from about 1 to about 3 months after formulation, but that such bitter flavor or aftertaste surprisingly develops in the packaged product over time.

It has also been discovered that many nutritional emulsions comprising calcium HMB are physically unstable, often resulting in the collection of excessive protein-containing and or other sediments at the bottom of the emulsion container, thus reducing nutrient availability as well as the effective shelf life of the product.

It has now also been found that these instability and or flavor issues can be minimized or eliminated by formulating with soluble protein that represents from about 50% to 100% by weight of the total protein in the nutritional emulsion, especially when the nutritional emulsion contains a weight ratio of the soluble protein to the calcium HMB of from about 5:1 to about 12:1. Soluble proteins of particular use in this regard include sodium caseinate and or whey protein concentrate, especially sodium caseinate.

## DETAILED DESCRIPTION OF THE DISCLOSURE

The nutritional emulsions of the present disclosure comprise calcium HMB and at least one ingredient, feature, or element to inhibit the development of bitter flavor or after taste and or to improve product stability over shelf life. The essential features of the nutritional emulsions, as well as some of the many optional variations and additions, are described in detail hereafter.

The term "calcium HMB" as used herein, unless otherwise specified, refers to the calcium salt of beta-hydroxy-beta-methybutyrate (also referred to as betadroxyl-3-methyl butyric acid, beta-hydroxy isovaleric acid, or HMB), which is most typically in a monohydrate form. All weights percentages, and concentrations as used herein to characterize calcium HMB are based on the weight of calcium HMB monohydrate, unless otherwise specified.

The term "nutritional emulsion" as used herein, unless otherwise specified, refers to liquid emulsions comprising fat, protein, and carbohydrate which are suitable for oral administration to a human.

The terms "fat" and "oil" as used herein, unless otherwise specified, are used interchangeably to refer to lipid materials derived or processed from plants or animals. These terms also include synthetic lipid materials so long as such synthetic materials are suitable for oral administration to humans.

The term "shelf stable" as used herein, unless otherwise specified, refers to a nutritional emulsion that remains commercially stable after being packaged and then stored at 18-24°C for at least 3 months, including from about 6 months to about 24 months, and also including from about 12 months to about 18 months.

The term "pH stable" as used herein, unless otherwise specified, means that the pH is resistant or at least more resistant to pH reductions due to a buffering effect of HMB.

The term "plastic" as used herein, unless otherwise specified, means food grade plastics approved by the U.S. Food and Drug Administration or other suitable regulatory group, some non-limiting examples of which include polyvinyl chlorides, polyethylene terephthalate, high density polyethylene, polypropylenes, polycarbonates, and so forth.

The terms "sterile", "sterilized" and "sterilization" as used herein, unless otherwise specified, refer to the reduction in transmissible agents such as fungi, bacteria, viruses, spore forms, and so forth, in food or on food grade surfaces to the extent necessary to render such foods suitable for human consumption. Sterilization processes may include various techniques involving the application of heat, peroxide or other chemicals, irradiation, high pressure, filtration, or combinations or variations thereof.

All percentages, parts and ratios as used herein, are by weight of the total composition, unless otherwise specified. All such weights as they pertain to listed ingredients are based on the active level and, therefore, do not include solvents or byproducts that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional emulsions of the present disclosure may also be substantially free of any optional or selected essential ingredient or feature described herein, provided that the remaining nutritional emulsion still contains all of the required ingredients or features as described herein. In this context, and unless otherwise specified, the term "substantially free" means that the selected nutritional emulsion contains less than a functional amount of the optional ingredient, typically less than about 0.5%, including less than about 0.1%, and also including zero percent, by weight of such optional or selected essential ingredient.

The nutritional emulsions and corresponding manufacturing methods of the present disclosure can comprise, consist of, or consist essentially of the essential elements of the disclosure as described herein, as well as any additional or optional element described herein or otherwise useful in nutritional emulsion formula applications.

## Product Form

The nutritional emulsions of the present disclosure are aqueous emulsions comprising fat, protein, and carbohydrate. These emulsions are flowable or drinkable liquids at from about 1 to about 25°C and are typically in the form of oil-in-water, water-in-oil, or complex aqueous emulsions, although such emulsions are most typically in the form of oil-in-water emulsions having a continuous aqueous phase and a discontinuous oil phase.

The nutritional emulsions may be and typically are shelf-stable. The nutritional emulsions typically contain up to about 95% by weight of water, including from about 50% to about 95%, also including from about 60% to about 90%, and also including from about 70% to about 85%, of water by weight of the nutritional emulsions.

The nutritional emulsions may be formulated with sufficient kinds and amounts of nutrients so as to provide a sole, primary, or supplemental source of nutrition, or to provide a specialized nutritional emulsion for use in individuals afflicted with specific diseases or conditions. These nutritional emulsions may thus have a variety of product densities, but most typically have a density greater than about 1.055 g/mL, including from 1.06 g/ml to 1.12 g/ml, and also including from about 1.085 g/ml to about 1.10 g/ml.

The nutritional emulsions may have a caloric density tailored to the nutritional needs of the ultimate user, although in most instances the emulsions comprise from about 100 to about 500 kcal/240 ml, including from about 150 to about 350 kcal/240 ml, and also including from about 200 to about 320 kcal/240 ml. These nutritional emulsions also comprise calcium HMB as described hereinafter, the amount of which most typically ranges from about 0.4 to about 3.0 g/240 ml, including from about 0.75 to about 2.0 g/240 ml, including about 1.5 g/240 ml.

The nutritional emulsion may have a pH ranging from about 3.5 to about 8, but are most advantageously in a range of from about 4.5 to about 7.5, including from about 5.5 to about 7.3, including from about 6.2 to about 7.2.

Although the serving size for the nutritional emulsion can vary depending upon a number of variables, a typical serving size ranges from about 100 to about 300 ml, including from about 150 to about 250ml, including from about 190 ml to about 240 ml.

## Macronutrients

The nutritional emulsions comprise fat, protein, and carbohydrate. Generally, any source of fat, protein, and carbohydrate that is known or otherwise suitable for use in nutritional products may also be suitable for use herein, provided that such macronutrients are also compatible with the essential elements of the nutritional emulsions as defined herein.

Although total concentrations or amounts of the fat, protein, and carbohydrates may vary depending upon the nutritional needs of the intended user, such concentrations or amounts most typically fall within one of the following embodied ranges, inclusive of any other essential fat. protein, and or carbohydrate ingredients as described herein.

Carbohydrate concentrations most typically range from about 5% to about 40%, including from about 7% to about 30%, including from about 10% to about 25%, by weight of the nutritional emulsion; fat concentrations most typically range from about 1% to about 30%, including from about 2% to about 15%, and also including from about 4% to about 10%, by weight of the nutritional emulsion; and protein concentrations most typically range from about 0.5% to about 30%, including from about 1% to about 15%, and also including from about 2% to about 10%, by weight of the nutritional emulsion.

The level or amount of carbohydrates, fats, and or proteins in the nutritional emulsions may also be characterized in addition to or in the alternative as a percentage of total calories in the nutritional compositions as set forth in the following table.

| Nutrient (% Calories) | Embodiment A | Embodiment B | Embodiment C |
| --- | --- | --- | --- |
| Carbohydrate | 1-98 | 10-75 | 30-50 |
| Fat | 1-98 | 20-85 | 35-55 |
| Protein | 1-98 | 5-70 | 15-35 |

Non-limiting examples of suitable fats or sources thereof for use in the nutritional emulsions described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

Non-limiting examples of suitable carbohydrates or sources thereof for use in the nutritional emulsions described herein may include maltodextrin, hydrolyzed or modified starch or cornstarch, glucose polymers, corn syrup, corn syrup solids, rice- derived carbohydrates, glucose, fructose, lactose, high fructose corn syrup, honey, sugar alcohols (e.g., maltitol, erythritol, sorbitol), and combinations thereof.

Non-limiting examples of suitable protein or sources thereof for use in the nutritional emulsions include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy) or combinations thereof. Non-limiting examples of such proteins include milk protein isolates, milk protein concentrates as described herein, casein protein isolates, whey protein, sodium or calcium caseinates, whole cow's milk, partially or completely defatted milk, soy protein isolates, soy protein concentrates, and so forth.

**Calcium HMB**

The nutritional emulsions comprise calcium HMB, which means that the emulsions are either formulated with the addition of calcium HMB, most typically as a monohydrate, or are otherwise prepared so as to contain calcium and HMB in the finished product. Any source of HMB is suitable for use herein provided that the finished product contains calcium and HMB, although such a source is preferably calcium HMB and is most typically added as such to the nutritional emulsion during formulation.

The term "added calcium HMB" as used herein means a calcium salt of HMB, most typically as monohydrate calcium salt of HMB, as the HMB source added to the nutritional emulsion.

Although calcium HMB monohydrate is the preferred source of HMB for use herein, other suitable sources may include HMB as the free acid, a salt, an anhydrous salt, an ester, a lactone, or other product forms that otherwise provide a bioavailable form of HMB from the nutritional emulsion. Non-limiting examples of suitable salts of HMB for use herein include HMB salts, hydrated or anhydrous, of sodium, potassium, magnesium, chromium, calcium, or other non-toxic salt form. Calcium HMB monohydrate is preferred and is commercially available from Technical Sourcing International (TSI) of Salt Lake City, Utah.

The concentration of calcium HMB in the nutritional emulsions may range up to about 10%, including from about 0.1% to about 8%, and also including from about 0.2% to about 5.0%, and also including from about 0.3% to about 3%, and also including from about 0.4% to about 1.5%, by weight of the nutritional emulsion.

**Soluble Protein**

The nutritional emulsions of the present disclosure may comprise selected amounts or ratios of soluble protein to improve product stability and minimize the development of bitter flavors and after taste during shelf life.

The soluble protein may represent from about 50% to 100%, including from 55% to 100%, including from about 60% to about 100%, including from about 60% to about 85%, including from about 60% to about 80%, and also including from about 65% to about 75%, by weight of the total protein in the nutritional emulsion. The concentration of soluble protein may range from at least about 0.5%, including from about 1% to about 26%, and also including from about 2% to about 15%, also including from about 3% to about 10%, and also including from about 4% to about 8%, by weight of the nutritional emulsion.

The amount of soluble protein included in the nutritional emulsions may also be characterized as a weight ratio of soluble protein to calcium HMB, wherein the nutritional emulsion includes a weight ratio of soluble protein to calcium HMB of at least about 3.0, including from about 4.0 to about 12.0, also including 6.1 to about 12, also including from about 7.0 to about 11.0, and also including from about 8.0 to about 10.0.

The term "soluble protein" as used herein, unless otherwise specified. refers to those proteins having a solubility of at least about 90% as measured in accordance with a Protein Solubility Measurement Test that includes the following steps: (1) suspend the protein at 2.00% (w/w) in water; (2) stir vigorously for one hour at 20°C to form a suspension; (3) remove an aliquot of the suspension, and determine protein concentration as total protein; (4) centrifuge the suspension at 31,000 x g and at 20°C for one hour; (5) determine the protein concentration in the supernatant (the soluble protein); and (6) express the soluble protein as a percentage of the total protein.

Any soluble protein source is suitable for use herein provided that it meets the solubility requirement as defined herein, some non-limiting examples of which include sodium caseinate (>95% solubility as determined by the Protein Solubility Measurement Test), whey protein concentrate (>90% solubility as determined by the Protein Solubility Measurement Test), and combinations thereof. Non-soluble proteins may of course also be included in the nutritional emulsions provided that the remaining soluble protein component is represented in accordance with the requirements as set forth herein.

Soluble protein suitable for use herein may also be characterized by the content of phosphoserine in the protein, wherein the soluble proteins in this context are defined as those proteins having at least about 100 mmoles, including from about 150 to 400 mmoles, including from about 200 to about 350 mmoles, and also including from about 250 to about 350 mmoles, of phosphoserine per kilogram of protein.

When the soluble protein is defined in terms of phosphoserine content, it has been found that the weight ratio of the soluble protein (with the defined phosphoserine content) to the calcium HMB may be at least about 3:1, including at least about 5:1, and also including at least about 7:1, and also including from about 9:1 to about 30:1. In this context, the proteins having the requisite content of phosphoserine are most typically in the form of monovalent caseinate salts such as sodium caseinate, potassium caseinate, and combinations thereof.

In one embodiment, the soluble protein may also be characterized by a mole ratio of monovalent caseinate phosphoserine to calcium HMB monohydrate of least about 0.2, including from about 0.2 to about 2.0, and also including from about 0.25 to 1.7.

It should be understood, however, that any phosphoserine-containing protein may be suitable for use herein provided that it has the requisite phosphoserine content and that the phosphoserine used in calculating the ratios are not bound, complexed, or otherwise attached to a polyvalent cation such as calcium or magnesium.

It should also be noted that alternative definitions as described herein for soluble proteins may include proteins that have little or no phosphoserine content, so that the soluble protein fraction of the compositions may include soluble protein with and/or without phosphoserine. The soluble protein for use herein may therefore be defined by any one or more of the soluble protein characterizations, separately or in combination.

The phosphoserine moieties within the protein may therefore be available for binding with the calcium released from the calcium HMB so that the above ratios of soluble protein to calcium HMB are the ratio of protein with phosphoserine moities that are unbound, unattached, or otherwise available to bind soluble calcium from the calcium HMB during formulation. It could be, for example, that a mixture of calcium caseinate and sodium caseinate are used in the composition but the ratio of proteins defined by a phosphoserine content to calcium HMB is calculated based on the protein fraction from the sodium caseinate and additionally any protein from the calcium caseinate fraction that is not bound to calcium.

### Soluble Calcium Binding Capacity

The nutritional emulsions may comprise a selected weight ratio of a soluble calcium binding capacity (SCBC) to the total soluble calcium in the emulsion to improve product stability and minimize the development over time of bitter flavors and after taste.

The ratio of the soluble calcium binding capacity (defined herein) to total soluble calcium of the nutritional emulsions is a weight ratio of at least about 2.3, including from about 2.3 to about 12.0, also including from about 3.0 to about 8.0, and also including from about 4.0 to about 6.5, wherein the ratio is determined in accordance with the following formulas:

$$Ratio = SCBC / [soluble\ calcium]$$

$$SCBC = (0.32 \times [soluble\ citrate] + 0.63\ [soluble\ phosphate] + 0.013 \times [soluble\ protein])$$

The weight ratio of SCBC to the concentration of total soluble calcium can be adjusted to minimize the concentration of unbound calcium in the nutritional emulsion, or to minimize the weight ratio of such unbound calcium to HMB in the emulsions, to improve product stability and reduce the development over time of bitter flavors and after tastes.

The nutritional emulsions of the present disclosure comprise calcium as a desirable ingredient in the nutritional emulsions suitable for use in developing or maintaining healthy muscle in targeted individuals. Some or all of the calcium may be provided by the addition of calcium HMB as described herein. Any other calcium source, however, may be used provided that such other source is compatible with the essential elements of the nutritional emulsions.

The concentration of calcium in the nutritional emulsions typically exceeds about 10 mg/L, and may also include concentrations of from about 25 mg/L to about 3000 mg/L, also including from about 50 mg/L to about 500 mg/L, and also including from about 100 mg/L to about 300mg/L.

To minimize the taste and stability issues in the nutritional emulsions, the calcium is formulated so as to minimize the extent to which the calcium is solubilized in the emulsions. As such, solubilized calcium concentrations in the nutritional emulsions may be less than about 900 mg/L, including less than about 700mg/L, also including from about 500mg/L to about 700mg/L, and also including from about 400mg/L to about 600 mg/L. In this context, the term "solubilized calcium" refers to free, ionized, or supernatant calcium in the nutritional emulsion as measured at 20°C.

The calcium in the nutritional emulsions may also be characterized by a ratio (on an equivalents basis) of solubilized citrate to solubilized calcium of not more than 5.0, including not more than 4.0, also including not more than 3.0, and also including from about 0.8 to about 3.0. In this context, the terms "solubilized citrate" and "solubilized calcium" refers to the equivalents of citrate and calcium cations, respectively, present in the supernatants of the nutritional emulsion as measured at 20°C.

The calcium component of the nutritional emulsion may also be characterized by a solubilized calcium level that represents less than 900 mg/L, including less than 700 mg/L, and also including less than 600 mg/L, and also including from 400 mg/L to 700 mg/L of the nutritional emulsion, wherein the weight ratio of calcium HMB to the solubilized calcium ranges from about 6 to about 15, including from about 6 to about 12, also including from about 6 to about 10, and also including from about 6 to about 8.

## Vitamin D

The nutritional emulsions of the present disclosure may further comprise vitamin D to help maintain healthy muscle in the targeted user. Vitamin D forms include Vitamin D2 (ergocalciferol) and Vitamin D3 (cholecalciferol) or other forms suitable for use in a nutritional product.

The amount of Vitamin D in the nutritional emulsion most typically ranges up to about 1000 IU, more typically from about 10 to about 600 IU, and more typically from about 50 to 400 IU, per serving of the nutritional emulsion.

## Optional Ingredients

The nutritional emulsions described herein may further comprise other optional ingredients that may modify the physical, chemical, hedonic or processing characteristics of the products or serve as pharmaceutical or additional nutritional components when used in the targeted population. Many such optional ingredients are known or otherwise suitable for use in other nutritional products and may also be used in the nutritional emulsions described herein, provided that such optional ingredients are safe and effective for oral administration and are compatible with the essential and other ingredients in the selected product form.

Non-limiting examples of such optional ingredients include preservatives, antioxidants, emulsifying agents, buffers, pharmaceutical actives, additional nutrients as described herein, colorants, flavors, thickening agents and stabilizers, and so forth.

The nutritional emulsions may further comprise vitamins or related nutrients, non-limiting examples of which include vitamin A, vitamin E, vitamin K, thiamine, riboflavin, pyridoxine, vitamin B12, carotenoids, niacin, folic acid, pantothenic acid, biotin, vitamin C, choline, inositol, salts, and derivatives thereof, and combinations thereof.

The nutritional emulsion may further comprise minerals, non-limiting examples of which include phosphorus, magnesium, iron, zinc, manganese, copper, sodium, potassium, molybdenum, chromium, selenium, chloride, and combinations thereof.

The nutritional emulsions may also include one or more masking agents to reduce or otherwise obscure the development of any residual bitter flavors and after taste in the emulsions over time. Suitable masking agents include natural and artificial sweeteners, sodium sources such as sodium chloride, and hydrocolloids, such as guar gum, xanthan gum, carrageenan, gellan gum, and combinations thereof. The amount of masking agent in the nutritional emulsion may vary depending upon the particular masking agent selected, other ingredients in the formulation, and other formulation or product target variables. Such amounts, however, most typically range from at least about 0.1%, including form about 0.15% to about 3.0%, and also including from about 0.18% to about 2.5%, by weight of the nutritional emulsion.

## Methods of Manufacture

The nutritional emulsions for use herein may be manufactured by any known or otherwise suitable method for making nutritional emulsions, including milk- based nutritional emulsions.

In one suitable manufacturing process, at least three separate slurries are prepared, including a protein-in-fat (PIF) slurry, a carbohydrate-mineral (CHO-MIN) slurry, and a protein-in-water (PIW) slurry. The PIF slurry is formed by heating and mixing the selected oils (e.g., canola oil, corn oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. Avicel, gellan, carrageenan), and calcium HMB or other HMB source. The resulting CHO-MIN slurry is held for 10 minutes with continued heat and agitation before adding additional minerals (e.g., potassium chloride, magnesium carbonate, potassium iodide, etc.), carbohydrates (e.g., frucotooligosaccharide, sucrose, corn syrup, etc.). The PIW slurry is then formed by mixing with heat and agitation the remaining protein (e.g., sodium caseinate, soy protein concentrate, etc.) into water.

The resulting slurries are then blended together with heated agitation and the pH adjusted to the desired range, typically from 6.6-7.0, after which the composition is subjected to high-temperature short-time (HTST) processing during which the composition is heat treated, emulsified and homogenized, and then allowed to cool. Water soluble vitamins and ascorbic acid are added, the pH is again adjusted to the desired range if necessary, flavors are added, and water is added to achieve the desired total solid level. The composition is then aseptically packaged to form an aseptically packaged nutritional emulsion, or the composition is added to retort stable containers and then subjected to retort sterilization to form retort sterilized nutritional emulsions.

The manufacturing processes for the nutritional emulsions may be carried out in ways other than those set forth herein without departing from the spirit and scope of the present invention. The present embodiments are, therefore, to be considered in all respects illustrative and not restrictive and that all changes and equivalents also come within the description of the present disclosure.

## Method of Use

The nutritional emulsions described herein are useful to provide supplement, primary, or sole sources of nutrition, and or to provide individuals one or more benefits as described herein. In accordance with such methods, the emulsions may be administered orally as needed to provide the desired level of nutrition, most typically in the form of one to two servings daily, in one or two or more divided doses daily, e.g., serving sizes typically ranging from about 100 to about 300 ml, including from about 150 to about 250ml, including from about 190 ml to about 240 ml, wherein each serving contains from about 0.4 to about 3.0 g, including from about 0.75 to about 2.0 g, including about 1.5 g, of calcium HMB per serving.

Such methods are further directed to provide the individual upon administration of such products, most typically after daily use over an extended period of time of from about 1 to about 6 months, including from about 1 to about 3 months, one or more of 1) to support maintenance of lean body mass, 2) to support of strength and or muscle strength, 3) to decrease protein breakdown and damage of muscle cells, and 4) to help with muscle recovery following exercise or other trauma, and 5) to reduce muscle protein breakdown following exercise.

Such methods are also helpful to achieve one or more of 1) to maintain and support lean body mass in elderly with sarcopenia, 2) to provide nutrition to support an active and independent lifestyle in individuals, especially in the elderly, 3) to support recovery of muscle strength, 4) to help rebuild muscle and regain strength, and 5) to improve strength, including muscle strength, and mobility.

## DATA

Nutritional emulsions A, B and C as referenced below are formulated, aseptically packaged, and evaluated for flavor. The nutritional emulsions are similar in composition and are prepared by similar methods. Each sample is an oil-in-water emulsion comprising fat, protein, carbohydrates, vitamins, and minerals, and includes 1.5 g of calcium HMB per each 240 ml of emulsion. The samples vary, however, in that each contains a different percentage of soluble protein by weight of total protein. The samples are evaluated 6 months after formulation and packaging using standard sensory methods (5 point scale), the results of which are summarized below.

| Sample | Soluble Protein Fraction (wt % of total protein) | Protein System (wt% of total protein) | Bitter Note | Soapy Note |
|---|---|---|---|---|
| A | 16.4 | 16.4% Na caseinate<br>53.6% acid casein<br>20% MPI, 10% SPI | 2.5 | 1.5 |
| B | 40 | 40% Na caseinate<br>30% Ca caseinate<br>20% MPI, 10% SPI | 1.0 | 1.0 |
| C | 70 | 70% Na caseinate<br>20% MPI, 10% SPI | 0.5 | 0.5 |

The data show a surprising correlation between the soluble protein fraction (e.g., Na caseinate) and the development of bitter and soapy sensory notes over time. The data also suggest, surprisingly, that the development of such undesirable sensory notes can be minimized or reduced by formulating with a majority of the total protein as soluble protein.

## EXAMPLES

The following examples illustrate specific embodiments and or features of the nutritional emulsions of the present disclosure. The examples are given solely for the purpose of illustration and are not to be construed as limitations of the present disclosure. All exemplified amounts are weight percentages based upon the total weight of the composition, unless otherwise specified.

The exemplified compositions are shelf stable nutritional emulsions prepared in accordance with the manufacturing methods described herein, such that each exemplified composition, unless otherwise specified, includes an aseptically processed embodiment and a retort packaged embodiment. These compositions are aqueous oil-in-water emulsions that are packaged in 240 ml plastic containers and remain physically stable for 12-18 months after formulation/packaging at storage temperatures ranging from 1-25°C. The packaged emulsions likewise remain pH stable over time and do not develop an excessively bitter flavor or aftertaste during the storage.

## Examples 1-4

Examples 1-4 illustrate nutritional emulsions of the present disclosure, the ingredients of which are listed in the table below. All ingredient amounts are listed as kilogram per 1000 kilogram batch of product, unless otherwise specified.

| Ingredient | Example 1 | Example 2 | Example 3 | Example 4 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 18.65 | 18.65 | 18.65 | 18.65 |
| Canola Oil | 27.5 | 27.5 | 27.5 | 27.5 |
| Sodium Caseinate | 26.68 | 26.68 | 26.68 | 26.68 |
| Soy Protein Concentrate | 14.05 | 14.05 | 14.05 | 14.05 |
| Corn Oil | 15.70 | 15.70 | 15.70 | 15.70 |
| Calcium HMB monohydrate | 6.00 | 6.5 | 7.0 | 4 |
| Whey Protein Concentrate | 3.50 | 3.50 | 3.50 | 3.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.030 | 0.050 |
| Vitamin A,D, E Premix | 0.0758 | 0.0758 | 0.0758 | 0.0758 |
| Water sol. Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 |
| Flavor | 3.3 | 3.3 | 3.3 | 3.3 |
| **Features** | | | | |
| Soluble protein/total protein (wt/wt) | 59% | 58% | 57% | 50% |
| , Soluble protein/calium HMB (wt/wt) | 6.2 | 5.6 | 5.1 | 7.5 |
| Solubilized calcium (wt%) | 0.045% | 0.049% | 0.053% | 0.070% |

(continued)

| Features | | | | |
|---|---|---|---|---|
| SCBC Solubilized calcium (wt/wt) | 5.5 | 5.0 | 4.5 | 3.0 |
| Solubilized citrate/solubilized calcium (equiv) | 3.5 | 3.0 | 2.5 | 1.5 |

**Examples 5-8**

Examples 5-8 illustrate nutritional emulsions of the present disclosure, the ingredients of which are listed in the table below All ingredient amounts are listed as kg per 1000 kg batch of product, unless otherwise specified.

| Ingredient | Example 5 | Example 6 | Example7 | Example 8 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 14.65 | 13.65 | 12.65 | 11.65 |
| Canola Oil | 27.5 | 27.5 | 27.5 | 27.5 |
| Sodium Caseinate | 30.68 | 31.68 | 32.68 | 33.68 |
| Soy Protein Concentrate | 14.05 | 14.05 | 14.05 | 14.05 |
| Corn Oil | 15.70 | 15.70 | 15.70 | 15.70 |
| Calcium HMB monohydrate | 6.00 | 6.5 | 7.0 | 7.5 |
| Whey Protein Concentrate | 3.50 | 3.50 | 3.50 | 3.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.050 | 0.050 |
| Vitamin A,D,E Premix | 0.0758 | 0.0758 | 0.0758 | 0.0758 |
| Water sol Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 |
| Flavor | 3.3 | 3.3 | 3.3 | 3.3 |
| Features | | | | |
| Soluble protein/total protein (wt/wt) | 63% | 64% | 65% | 66% |
| Soluble protein/calcium HMB (wt/wt) | 6.6 | 6.2 | 5.8 | 5.0 |

(continued)

| Features | | | | |
|---|---|---|---|---|
| Solubilized calcium (wt%) | 0.045% | 0.049% | 0.053% | 0.070% |
| SCBC / Solubilized calcium (wt/wt) | 5.5 | 5.0 | 4.5 | 3.0 |
| Solubilized citrate/solubilized calcium (equiv) | 3.5 | 3.0 | 2.5 | 1.5 |

**Examples 9-12**

Examples 9-12 illustrate nutritional emulsions of the present disclosure, the ingredients of which are listed in the table below. All ingredient amounts are listed as kilogram per 1000 kilogram batch of product, unless otherwise specified.

| Ingredient | Example 9 | Example 10 | Example 11 | Example 12 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 0.00 | 0.00 | 8.65 | 10.65 |
| Canola Oil | 27.5 | 27.5 | 27.5 | 27.5 |
| Sodium Caseinate | 45.33 | 45.33 | 36.68 | 34.68 |
| Soy Protein Concentrate | 0.00 | 0.00 | 12.05 | 9.05 |
| Corn Oil | 15.70 | 15.70 | 15.70 | 15.70 |
| Calcium HMB monohydrate | 6.0 | 6.5 | 7.0 | 8.0 |
| Whey Protein Concentrate | 17.55 | 17.55 | 5.50 | 8.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.050 | 0.050 |
| Vitamin A,D, E Premix | 0.0758 | 0.0758 | 0.0758 | 0.0758 |
| Water sol. Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 |
| Flavor | 3.3 | 3.3 | 3.3 | 3.3 |
| Features | | | | |
| Soluble protein/total protein (wt/wt) | 94% | 93% | 71% | 73% |

(continued)

| Features | | | | |
|---|---|---|---|---|
| Soluble protein/calcium HMB (wt/wt) | 9.8 | 9.0 | 6.4 | 5.1 |
| Solubilized calcium (wt%) | 0.045% | 0.050% | 0.058% | 0.070% |
| SCBC / Solubilized calcium (wt/wt) | 10 | 8.8 | 5.9 | 3.8 |
| Solubilized citrate/solubilized calcium (equiv) | 3.8 | 3.4 | 2.9 | 1.5 |

## Examples 13-16

Examples 13-16 illustrate nutritional emulsions of the present disclosure, the ingredients of which are listed in the table below. All ingredients amounts are listed as kilogram per 1000 kilogram batch of product, unless otherwise specified.

| Ingredient | Example 13 | Example 14 | Example 15 | Example 16 |
|---|---|---|---|---|
| Water | Q.S | Q.S. | Q.S. | Q.S. |
| Sucrose | 96.05 | 96.05 | 96.05 | 96.05 |
| Maltodextrin DE 5 | 16.46 | 16.46 | 16.46 | 16.46 |
| Milk Protein Concentrate | 18.95 | 0.00 | 8.95 | 25.00 |
| Soy Oil | 13.31 | 13.31 | 13.31 | 13.31 |
| Fructooligosaccharides | 8.69 | 8.69 | 8.69 | 8.69 |
| Soy Protein Concentrate | 13.80 | 0.00 | 10.80 | 5.92 |
| Canola Oil | 5.32 | 5.32 | 5.32 | 5.32 |
| Sodium Caseinate | 25.64 | 58.39 | 61.39 | 28.00 |
| Corn Oil | 11.70 | 11.70 | 11.70 | 11.70 |
| Calcium HMB monohydrate | 6.70 | 7.00 | 2.50 | 5.00 |
| Dietary Fiber | 4.51 | 4.51 | 4.51 | 4.51 |
| Whey Protein Concentrate | 3.44 | 3.44 | 13.44 | 2.92 |
| Potassium Citrate | 4.48 | 4.48 | 4.48 | 4.48 |
| Flavor | 2.00 | 2.00 | 2.00 | 2.00 |
| Magnesium Phosphate | 2.75 | 2.75 | 2.75 | 2.75 |
| Lecithin | 1.50 | 1.50 | 1.50 | 1.50 |
| Di sodium Phosphate Dihyd | 0.436 | 0.436 | 0.436 | 0.436 |
| Potassium Phosphate Dibasic | 0.556 | 0.556 | 0.556 | 0.556 |
| Sodium Chloride | 0.498 | 0.498 | 0.498 | 0.498 |
| Choline Chloride | 0.480 | 0.480 | 0.480 | 0.480 |
| Ascorbic Acid | 0.465 | 0.465 | 0.465 | 0.465 |
| Carrageenan | 0.300 | 0.300 | 0.300 | 0.300 |
| Trace/Ultra Trace minerals | 0.420 | 0.420 | 0.420 | 0.420 |
| Potassium Chloride | 0.698 | 0.698 | 0.698 | 0.698 |
| Potassium Hydroxide | 0.321 | 0.321 | 0.321 | 0.321 |
| L-carnitine | 0.180 | 0.180 | 0.180 | 0.180 |
| Water soluble Vitamin Premix | 0.07269 | 0.07269 | 0.07269 | 0.07269 |

(continued)

| Ingredient | Example 13 | Example 14 | Example 15 | Example 16 |
|---|---|---|---|---|
| Vitamin DEK premix | 0.128 | 0.128 | 0.128 | 0.128 |
| Gellan Gum | 0.050 | 0.050 | 0.050 | 0.050 |
| Vitamin A Palmitate | 0.008245 | 0.008245 | 0.008245 | 0.008245 |
| Vitamin D3 | 0.000399 | 0.000399 | 0.000399 | 0.000399 |
| Potassium Iodide | 0.000194 | 0.000194 | 0.000194 | 0.000194 |
| **Features** | | | | |
| Soluble protein/total protein (wt/wt) | 58% | 95% | 80% | 61% |
| Soluble protein/calcium HMB (wt/wt) | 5.4 | 8.4 | 30 | 15 |
| Solubilized calcium (wt%) | 0.050% | 0.060% | 0.080% | 0.055% |
| SCBC / Solubilized calcium (wt/wt) | 4.4 | 9.7 | 8.8 | 4.9 |
| Solubilized citrate/solubilized calcium (equiv) | 1.3 | 3.1 | 2.7 | 2.9 |

**Examples 17-20**

Examples 17-20 illustrate nutritional emulsions of the present disclosure, the ingredients of which are listed in the table below. All ingredient amounts are listed as kilogram per 1000 kilogram batch of product, unless otherwise specified.

| Ingredient | Example 17 | Example 18 | Example 19 | Example 20 |
|---|---|---|---|---|
| Water | Q.S | Q.S. | Q.S. | Q.S. |
| Sucrose | 96.05 | 96.05 | 96.05 | 96.05 |
| Maltodextrin DE 5 | 16.46 | 16.46 | 16.46 | 16.46 |
| Milk Protein Concentrate | 24.98 | 0.00 | 25.00 | 10.00 |
| Soy Oil | 13.31 | 13.31 | 13.31 | 13.31 |
| Fructooligosaccharides | 8.69 | 8.69 | 8.69 | 8.69 |
| Soy Protein Concentrate | 13.64 | 0.00 | 5.87 | 10.64 |
| Canola Oil | 5.32 | 5.32 | 5.32 | 5.32 |
| Sodium Caseinate | 25.64 | 58.39 | 61.39 | 28.00 |
| Corn Oil | 11.70 | 11.70 | 11.70 | 11.70 |
| Calcium HMB monohydrate | 6.50 | 3.5 | 4.25 | 7.5 |
| Dietary Fiber | 4.51 | 4.51 | 4.51 | 4.51 |
| Whey Protein Concentrate | 3.40 | 17.04 | 6.87 | 6.40 |
| Potassium Citrate | 4.48 | 4.48 | 4.48 | 4.48 |
| Flavor | 2.00 | 2.00 | 2.00 | 2.00 |
| Magnesium Phosphate | 2.75 | 2.75 | 2.75 | 2.75 |
| Lecithin | 1.50 | 1.50 | 1.50 | 1.50 |
| Di sodium Phosphate Dihyd | 0.436 | 0.436 | 0.436 | 0.436 |
| Potassium Phosphate Dibasic | 0.556 | 0.556 | 0.556 | 0.556 |
| Sodium Chloride | 0.498 | 0.498 | 0.498 | 0.498 |
| Choline Chloride | 0.480 | 0.480 | 0.480 | 0.480 |

(continued)

| Ingredient | Example 17 | Example 18 | Example 19 | Example 20 |
|---|---|---|---|---|
| Ascorbic Acid | 0.465 | 0.465 | 0.465 | 0.465 |
| Carrageenan | 0.300 | 0.300 | 0.300 | 0.300 |
| Trace/Ultra Trace minerals | 0.420 | 0.420 | 0.420 | 0.420 |
| Potassium Chloride | 0.698 | 0.698 | 0.698 | 0.698 |
| Potassium Hydroxide | 0.321 | 0.321 | 0.321 | 0.321 |
| L-carnitine | 0.180 | 0.180 | 0.180 | 0.180 |
| Water soluble Vitamin Premix | 0.07269 | 0.07269 | 0.07269 | 0.07269 |
| Vitamin DEK premix | 0.128 | 0.128 | 0.128 | 0.128 |
| Gellan Gum | 0.050 | 0.050 | 0.050 | 0.050 |
| Vitamin A Palmitate | 0.008245 | 0.008245 | 0.008245 | 0.008245 |
| Vitamin D3 | 0.000399 | 0.000399 | 0.000399 | 0.000399 |
| Potassium Iodide | 0.000194 | 0.000194 | 0.000194 | 0.000194 |
| Features | | | | |
| Soluble protein/total protein (wt/wt) | 56% | 94% | 74% | 68% |
| Soluble protein/calcium HMB (wt/wt) | 5.8 | 20 | 17 | 5.0 |
| Solubilized calcium (wt%) | 0.057% | 0.085% | 0.079% | 0.060% |
| SCBC / Solubilized calcium (wt/wt) | 2.9 | 7.9 | 6.8 | 4.7 |
| Solubilized citrate/solubilized calcium (equiv) | 3.0 | 0.9 | 1.5 | 2.2 |

**Claims**

1. A nutritional emulsion comprising fat, carbohydrate, protein, and calcium beta-hydroxy-beta-methylbutyrate (HMB) wherein a soluble protein represents from 50% to 100% by weight of total protein in the emulsion.

2. The nutritional emulsion of claim 1 wherein the soluble protein represents from about 55% to 100% by weight of the total protein and includes phosphoserine-containing protein having at least about 100 mmoles of phosphoserine per kilogram of phosphoserine-containing protein.

3. The nutritional emulsion of claim 2 wherein the soluble protein represents from about 60% to 85% by weight of the total protein.

4. The nutritional emulsion of claim 1 wherein the soluble protein includes at least one protein selected from the group consisting of sodium caseinate, whey protein concentrate, and combinations thereof.

5. The nutritional emulsion of claim 1 wherein the emulsion comprises from about 0.3% to about 1.5% by weight of calcium beta-hydroxy-beta-methylbutyrate (HMB).

6. The nutritional emulsion of claim 1 wherein the emulsion is packaged in a hermetically sealed container and is shelf stable at a temperature of from 18° C to 24° C for at least about 3 months.

7. A nutritional emulsion comprising fat, carbohydrate, protein, and calcium beta-hydroxy-beta-methylbutyrate (HMB) wherein soluble protein comprises from about 50% to 100% by weight of total protein and the emulsion has a weight ratio of soluble protein to calcium beta-hydroxy-beta-methylbutyrate (HMB) of at least about 5:1.

8. The nutritional emulsion of claim 7 wherein the soluble protein represents from about 55% to 100% by weight of the total protein and includes phosphoserine-containing protein having at least about 100 mmoles of phosphoserine per kilogram of phosphoserine-containing protein.

9. The nutritional emulsion of claim 7 wherein the soluble protein represents from about 60% to 85% by weight of the total protein.

10. The nutritional emulsion of claim 7 wherein the soluble protein includes at least one protein selected from the group consisting of sodium caseinate, whey protein concentrate, and combinations thereof.

11. The nutritional emulsion of claim 7 wherein the soluble protein comprises sodium caseinate.

12. The nutritional emulsion of claim 7 wherein the concentration of soluble protein is from about 1% to about 26% by weight of the nutritional emulsion.

13. The nutritional emulsion of claim 7 wherein the emulsion comprises from about 0.3% to about 1.5% by weight of calcium beta-hydroxy-beta-methylbutyrate (HMB).

14. The nutritional emulsion of claim 7 wherein the weight ratio of soluble protein to calcium beta-hydroxy-beta-methylbutyrate (HMB) is from about 7:1 to about 10:1.

15. The nutritional emulsion of claim 7 wherein the emulsion is packaged in a hermetically sealed container and is shelf stable at a temperature of from 18°C to 24°C for at least about 3 months.

**Patentansprüche**

1. Eine Ernährungsemulsion umfassend Fett, Kohlenhydrate, Protein und Kalzium beta-Hydroxy-beta-methylbutyrat (HMB), worin ein lösliches Protein von 50 bis 100 Gewichtsprozent des Gesamtproteins in der Emulsion ausmacht.

2. Die Ernährungsemulsion gemäß Anspruch 1, worin das lösliche Protein von ungefähr 55 bis 100 Gewichtsprozent des Gesamtproteins ausmacht und Phosphoserin-enthaltendes Protein mit mindestens ungefähr 100 mmol Phosphoserin pro Kilogramm Phosphoserin-enthaltendem Protein einschließt.

3. Die Ernährungsemulsion gemäß Anspruch 2, worin das lösliche Protein von ungefähr 60 bis 85 Gewichtsprozent des Gesamtproteins ausmacht.

4. Die Ernährungsemulsion gemäß Anspruch 1, worin das lösliche Protein mindestens ein Protein gewählt aus der Gruppe bestehend aus Natriumcaseinat, Molkenproteinkonzentrat, und Kombinationen daraus einschließt.

5. Die Ernährungsemulsion gemäß Anspruch 1, worin die Emulsion von ungefähr 0,3 bis ungefähr 1,5 Gewichtsprozent von Kalzium beta-Hydroxy-beta-methylbutyrat (HMB) umfasst.

6. Die Ernährungsemulsion gemäß Anspruch 1, worin die Emulsion in einem hermetisch versiegelten Container verpackt ist und bei einer Temperatur von 18°C bis 24°C für mindestens 3 Monate lagerungsfähig ist.

7. Eine Ernährungsemulsion umfassend Fett, Kohlenhydrate, Protein und Kalzium beta-Hydroxy-beta-methylbutyrat (HMB), worin lösliches Protein von ungefähr 50 bis 100 Gewichtsprozent des Gesamtproteins umfasst, und die Emulsion ein Gewichtsverhältnis von löslichem Protein zu Kalzium beta-Hydroxy-beta-methylbutyrat (HMB) von mindestens ungefähr 5:1 hat.

8. Die Ernährungsemulsion gemäß Anspruch 7, worin das lösliche Protein von ungefähr 55 bis 100 Gewichtsprozent des Gesamtproteins ausmacht und Phosphoserin-enthaltendes Protein mit mindestens ungefähr 100 mmol Phosphoserin pro Kilogramm Phosphoserin-enthaltendem Protein einschließt.

9. Die Ernährungsemulsion gemäß Anspruch 7, worin das lösliche Protein von ungefähr 60 bis 85 Gewichtsprozent des Gesamtproteins ausmacht.

10. Die Ernährungsemulsion gemäß Anspruch 7, worin das lösliche Protein mindestens ein Protein gewählt aus der Gruppe bestehend aus Natriumcaseinat, Molkenproteinkonzentrat, und Kombinationen daraus einschließt.

**11.** Die Ernährungsemulsion gemäß Anspruch 7, worin das lösliche Protein Natriumcaseinat umfasst.

**12.** Die Ernährungsemulsion gemäß Anspruch 7, worin die Konzentration von löslichem Protein von ungefähr 1 bis ungefähr 26 Gewichtsprozent der Ernährungsemulsion beträgt.

**13.** Die Ernährungsemulsion gemäß Anspruch 7, worin die Emulsion von ungefähr 0,3 bis ungefähr 1,5 Gewichtsprozent Kalzium beta-Hydroxy-beta-methylbutyrat (HMB) umfasst.

**14.** Die Ernährungsemulsion gemäß Anspruch 7, worin das Gewichtsverhältnis von löslichem Protein zu Kalzium beta-Hydroxy-beta-methylbutyrat (HMB) von ungefähr 7:1 bis ungefähr 10:1 ist.

**15.** Die Ernährungsemulsion gemäß Anspruch 7, worin die Emulsion in einem hermetisch versiegelten Container verpackt ist und bei einer Temperatur von 18°C bis 24°C für mindestens 3 Monate lagerungsfähig ist.

**Revendications**

**1.** Emulsion nutritionnelle comprenant des graisses, des glucides, des protéines et du bêta-hydroxy-bêta-méthylbutyrate de calcium (HMB) où les protéines solubles représentent de 50 % à 100 % en poids des protéines totales dans l'émulsion.

**2.** Emulsion nutritionnelle selon la revendication 1 où les protéines solubles représentent d'environ 55 % à 100 % en poids des protéines totales et incluent des protéines contenant de la phosphosérine ayant au moins environ 100 mmoles de phosphosérine par kilogramme de protéines contenant de la phosphosérine.

**3.** Emulsion nutritionnelle selon la revendication 2 où les protéines solubles représentent d'environ 60 % à 85 % en poids des protéines totales.

**4.** Emulsion nutritionnelle selon la revendication 1 où les protéines solubles incluent au moins une protéine choisie dans le groupe consistant en le caséinate de sodium, un concentré de protéines du petit lait et leurs combinaisons.

**5.** Emulsion nutritionnelle selon la revendication 1 où l'émulsion comprend d'environ 0,3 % à environ 1,5 % en poids de bêta-hydroxy-bêta-méthylbutyrate de calcium (HMB).

**6.** Emulsion nutritionnelle selon la revendication 1 où l'émulsion est conditionnée dans un récipient fermé hermétiquement et est stable au stockage à une température de 18°C à 24°C pendant au moins environ 3 mois.

**7.** Emulsion nutritionnelle comprenant des graisses, des glucides, des protéines et du bêta-hydroxy-bêta-méthylbutyrate de calcium (HMB) où les protéines solubles comprennent d'environ 50 % à 100 % en poids des protéines totales et l'émulsion a un rapport en poids des protéines solubles au bêta-hydroxy-bêta-méthylbutyrate de calcium (HMB) d'au moins environ 5:1.

**8.** Emulsion nutritionnelle selon la revendication 7 où les protéines solubles représentent d'environ 55 % à 100 % en poids des protéines totales et incluent des protéines contenant de la phosphosérine ayant au moins environ 100 mmoles de phosphosérine par kilogramme de protéines contenant de la phosphosérine.

**9.** Emulsion nutritionnelle selon la revendication 7 où les protéines solubles représentent d'environ 60 % à 85 % en poids des protéines totales.

**10.** Emulsion nutritionnelle selon la revendication 7 où les protéines solubles incluent au moins une protéine choisie dans le groupe consistant en le caséinate de sodium, un concentré de protéines du petit lait et leurs combinaisons.

**11.** Emulsion nutritionnelle selon la revendication 7 où les protéines solubles comprennent du caséinate de sodium.

**12.** Emulsion nutritionnelle selon la revendication 7 où la concentration des protéines solubles est d'environ 1 % à environ 26 % en poids de l'émulsion nutritionnelle.

**13.** Emulsion nutritionnelle selon la revendication 7 où l'émulsion comprend d'environ 0,3 % à environ 1,5 % en poids de bêta-hydroxy-bêta-méthylbutyrate de calcium (HMB).

14. Emulsion nutritionnelle selon la revendication 7 où le rapport en poids des protéines solubles au bêta-hydroxy-bêta-méthylbutyrate de calcium (HMB) est d'environ 7:1 à environ 10:1.

15. Emulsion nutritionnelle selon la revendication 7 où l'émulsion est conditionnée dans un récipient fermé hermétiquement et est stable au stockage à une température de 18°C à 24°C pendant au moins environ 3 mois.